(19) **Europäisches Patentamt**

**European Patent Office**

**Office européen des brevets**

(11) **EP 1 593 707 A1**

(12) **EUROPÄISCHE PATENTANMELDUNG**

(43) Veröffentlichungstag:
**09.11.2005  Patentblatt 2005/45**

(51) Int Cl.[7]: **C08K 5/372**, C08G 75/14,
C08J 3/24, C07C 319/22,
C07C 323/30

(21) Anmeldenummer: **05006050.8**

(22) Anmeldetag: **19.03.2005**

(84) Benannte Vertragsstaaten:
**AT BE BG CH CY CZ DE DK EE ES FI FR GB GR
HU IE IS IT LI LT LU MC NL PL PT RO SE SI SK TR**
Benannte Erstreckungsstaaten:
**AL BA HR LV MK YU**

(30) Priorität: **31.03.2004  DE 102004015627**

(71) Anmelder: **LANXESS Deutschland GmbH
51369 Leverkusen (DE)**

(72) Erfinder:
• **Weidenhaupt, Hermann-Josef, Dr.
50259 Pulheim (DE)**
• **Buding, Hartmuth, Dr.
52445 Titz (DE)**
• **Hahn, Josef, Prof.Dr.
50996 Köln (DE)**
• **Mihalyi, Andreas, Dr.
50374 Erftstadt (DE)**
• **Zettl, Uwe, Dr.
50354 Hürth (DE)**

(54) **Neue Vulkanisiermittel auf Basis von organischen Schwefel-Stickstoff-Verbindungen für ungesättigte Kautschuke und deren Mischungen**

(57)    Die Erfindung betrifft neue organische Schwefel-Stickstoff-Verbindungen, deren Herstellung und Verwendung als Vulkanisiermittel für ungesättigte Kautschuke und deren Mischungen.

EP 1 593 707 A1

**Beschreibung**

Die Erfindung betrifft neue organische Schwefel-Stickstoff-Verbindungen, deren Herstellung und Verwendung als Vulkanisiermittel für ungesättigte Kautschuke und deren Mischungen.

Schwefel ist auch heute noch das gebräuchlichste Vulkanisationsmittel für ungesättigte Kautschuke, wie z. B. Naturkautschuk (NR), Isoprenkautschuk (IR), Butadienkautschuk (BR) und Styrol-Butadien-Kautschuk (SBR). Bei der Herstellung von Weichgummi verwendet man ca. 0,25 bis 5,0 Gew.-Teile Schwefel, bezogen auf 100 Gew.-Teile Kautschuk. Die effektiv angewendete Menge an Schwefel ist abhängig von der gewählten Menge an Vulkanisationsbeschleuniger, was letztlich durch die gewünschten Vulkanisateigenschaften bestimmt wird.

Sehr häufig gebrauchte Vulkanisiersysteme sind das konventionelle und das semi-effiziente Vulkanisiersystem. Das konventionelle Vulkanisiersystem ist reich an Schwefel und arm an Vulkanisationsbeschleuniger, wohingegen das semi-effiziente Vulkanisiersystem eine mittlere Dosierung an Schwefel und Vulkanisationsbeschleuniger aufweist. Dem Fachmann sind die typischen Dosierungen bekannt. Sie sind z.B. beschrieben in W. Hofmann, Kautschuk-Technologie, Genter Verlag, Stuttgart, 1980, S. 64 und 254-255. Konventionelle Vulkanisiersysteme ergeben dynamisch gut belastbare Vulkanisate, wobei jedoch diese sehr alterungs- und reversionsempfindlich sind. Semi-effiziente Vulkanisiersysteme ergeben üblicherweise dynamisch weniger gut belastbare Vulkanisate, dafür sind sie aber etwas alterungs- und reversionsstabiler.

Unter Reversion versteht man eine unter Temperatureinwirkung in Abwesenheit von Sauerstoff stattfindende Netzbrückenumlagerung, die zu einer Verschlechterung der Gebrauchseigenschaften des Vulkanisats führt und somit unerwünscht ist (anaerobe Alterung). Reversion findet unabdingbar einmal bei der Vulkanisation von sehr dicken und voluminösen Bauteilen statt, z.B. bei LKW-Reifen und Fendern. Die physikalische Erklärung hierfür ist einfach: Wenn die innenliegenden Kautschukmischungs-volumina durch den über die heiße Vulkanisationsform eingebrachten Wärmefluß gerade richtig vulkanisiert sind, so sind die unmittelbar an der heißen Vulkanisationsform anliegenden Kautschukmischungsvolumina naturgemäß bereits übervulkanisiert. Zum anderen findet während des Gebrauchs dieser Gummiteile bei lang andauernder, intensiver dynamischer Belastung durch mangelnde Hysterese (vgl. Walkarbeit des Reifens) eine Temperaturerhöhung (heat build-up) des Gummiteils statt, was eine Reversion des Vulkanisats nach sich zieht. Die Reversion kann in einem solchen Ausmaß stattfinden, dass sie sogar zur Zerstörung und damit zum Ausfall des Vulkanisats führt.

In den letzten Jahren wurden einige Reversionsschutzmittel-Spezialitäten bekannt, die die Reversion entweder durch Einbau von thermisch stabilen, praktisch nicht zur Reversion befähigten Netzbrücken minimieren (vgl. EP-A 530 590) oder die nach bereits erfolgter Reversion die gebrochenen, klassischen Netzstellen durch andere stabilere ersetzen (vgl. R.N. Datta und W.F. Helt, Rubber World, August 1997, S. 24 ff).

Kommerziell verfügbare Reversionsschutzmittel-Spezialitäten sind z.B. das Dinatriumsalz von Hexamethylen-1,6-dithiosulfat-dihydrat und 1,3-Bis(citraconimido-methyl)-benzol.

Allgemeiner Nachteil dieser kommerziellen Reversionsschutzmittel-Spezialitäten ist ihr relativ hoher Preis, der einmal durch nicht in großen Mengen verfügbaren Edukten sowie durch die schwierige und aufwendige Herstellung dieser Produkte bedingt ist, wodurch eine breite Anwendung in der unter stetigem Kostenreduzierungsdruck stehenden kautschukverarbeitenden Industrie, insbesondere in der Reifenindustrie, verhindert wird. Ein spezifischer Nachteil des Dinatrium-Hexamethylen-1,6-dithiosulfat-dihydrat ist seine aufwendige Lieferform. Aufgrund seines Salzcharakters muss es im Hinblick auf eine gute Einmischbarkeit sehr fein gemahlen werden, was andererseits wieder aus arbeitshygienischer Sicht eine Beölung des Pulvers zur Staubunterdrückung nach sich zieht.

Ein spezifischer Nachteil von 1,3-Bis(citraconimido-methyl)-benzol ist, dass es im Vulkanisat erst dann, und nur dann, wirksam werden kann, wenn bei dem mit Schwefel vernetzten ungesättigten Kautschuk die Reversion bereits eingesetzt hat und so konjugierte Olefine entstanden sind, die ihrerseits in einer Nachvernetzung mit dem Citraconderivat (via Diels-Alder-Reaktion) zu einem neuen, jetzt aber andersartigen Netzwerk abreagieren können.

Ein Nachteil, der in den Vulkanisiermitteln der EP 530 590 gesehen werden kann, ist ihr hohes Molekulargewicht, verglichen mit der eigentlich vernetzend wirkenden Spezies.

Polymere Alkylen-Schwefel- bzw. Arylen-Schwefel-Stickstoff-Verbindungen sind schon lange bekannt. So findet das Produkt Poly-Phenylen-Sulfid (PPS) Verwendung als Thermoplast. Eine Verwendung als Vulkanisiermittel für Kautschuk ist nicht bekannt. Eigene Versuche belegen, s. Beispiel 9, dass PPS eine schwarze Naturkautschukmischung nicht vernetzt. Polymere Alkylen-Schwefel-Verbindungen werden auch beschrieben. Abhängig von der Schwefelkettenlänge kann eine Vernetzung von ungesättigten Kautschuken stattfinden. Die hohe Vernetzerdosierung lässt aber auf einen Einbau mehrerer Alkylen-Schwefel-Einheiten pro Netzstelle schließen, dies bedeutet damit eine unbefriedigende Netzstellenausbeute.

Üblicherweise werden in breitem Maße die aus ungesättigten Kautschuken hergestellten Vulkanisate nur - wie erwähnt mit Schwefel und Beschleuniger als Vulkanisiermittel hergestellt, d.h. also ohne Agenzien, die die Reversion verhindern oder vermindern. Die Eigenschaften von klassisch mit konventionellen und semi-effizienten Vulkanisiersystemen hergestellten Kautschukvulkanisaten sind aber verbesserungswürdig. Es besteht somit ein Bedarf nach einem Vulkanisiermittel für ungesättigte Kautschuke, das überwiegend auf in großen Mengen leicht und kostengünstig verfügbaren Bausteinen basiert, technisch leicht herstellbar ist, und das den zum Ausblühen neigenden kristallinen Schwefel in einem Vulkanisiersystem ganz oder teilweise ersetzen kann und zu Vulkanisaten mit verbesserter Reversionsstabilität, insbesondere nach Überheizung führt.

Die Aufgabe wurde überraschenderweise gelöst durch Vulkanisation einer ungesättigten Kautschukmischung mit speziellen neuen organischen Schwefel-Stickstoff-Verbindungen.

Gegenstand der Erfindung sind daher organische Schwefel-Stickstoff-Verbindungen der Formel (I)

$$R^1R^2 N(-S_x-R^3-S_x-N R^4)_n-S_x-R^3-S_x-NR^5R^6,$$

worin

| | |
|---|---|
| $R^1$, $R^2$, $R^5$ und $R^6$ | gleich oder verschieden sind und für Wasserstoff, Methyl, Ethyl, n-Butyl, iso-Butyl, t-Butyl, Hexyl, Cyclohexyl, Octyl, Phenyl oder Benzyl stehen, |
| $R^4$ | Methyl, Ethyl, n-Butyl, iso-Butyl, t-Butyl, Hexyl, Cyclohexyl, Octyl, Phenyl oder Benzyl bedeutet, |
| n | eine ganze Zahl von 1 bis 50, bevorzugt von 1 bis 10 ist, mit der Maßgabe, dass n = 0 ist, wenn $R^1$, $R^2$, $R^5$ oder $R^6$ ungleich Wasserstoff ist, |
| $R^3$ | für Phenylen, Methylen, Ethylen, Butylen oder Hexandiyl steht und |
| x | eine ganze Zahl von 2 bis 6 darstellt. |

Bevorzugt steht

| | |
|---|---|
| $R^1$, $R^2$, $R^5$ und $R^6$ | für Methyl, Ethyl, t-Butyl, Benzyl, Cyclohexyl, |
| $R^4$ | für Methyl, Ethyl, t-Butyl, Benzyl, Cyclohexyl, |
| $R^3$ | für Phenylen, Hexandiyl und |
| x | für ganze Zahlen von 2 bis 4. |

Ein weiterer Gegenstand der Erfindung ist die Herstellung der organischen Schwefel-Stickstoff-Verbindungen der obigen Formel und deren Verwendung als Vulkanisiermittel für ungesättigte Kautschuke.

Die erfindungsgemäßen organischen Schwefel-Stickstoff-Verbindungen der obigen Formel werden erhalten durch Umsetzung der entsprechenden Aminkomponente mit entsprechenden Bis(dichloro-polysulfonyl)arylenen oder -alkylenen, z.B. 1,4-Bis(chlor-disulfanyl)benzol oder 1,6-Bis(Chlortrisulfanyl)hexan in inerten Lösungsmitteln bei Raumtemperatur. Das ausgefallene Salz wird abfiltriert. Die organische Phase wird mit Wasser salzfrei gewaschen, getrocknet und anschließend wird das Lösungsmittel abdestilliert. Die neuen Verbindungen können als Sumpfprodukt ohne weitere Aufarbeitung für die Vulkanisation eingesetzt werden. Des Weiteren können die Schwefel-Stickstoff-Verbindungen durch (Polymerabbau) der entsprechenden Poly (polythio)alkane erhalten werden (s. Beispiel 8).

Die einzusetzenden Schwefelchlorverbindungen können selbstverständlich ohne weitere Vorreinigung direkt aus ihrer Synthese eingesetzt werden. Sie erhalten dann noch oligomere Anteile. Der oligomere Anteil kann bis zu 80 Gew.-%, bezogen auf Ausgangsverbindung, bevorzugt bis zu 50 Gew.-% betragen.

Bei Verwendung von sekundären Aminen werden zwangsläufig immer monomere organische Amin-Vernetzer erhalten. Bei Verwendung von primären Aminen können in Abhängigkeit der eingesetzten Aminüberschusses auch oligomere organische Strukturen erhalten werden. Bei geringem Aminüberschuss sollten diese überwiegen. Als primäre Amine können eingesetzt werden z.B. Methylamin, Ethylamin, Propylamin, Isobutylamin, n-Butylamin, t-Butylamin, Hexylamin, Cyclohexylamin, Benzylamin, Phenylamin oder Octylamin. Als sekundäre Amine können z.B. eingesetzt werden Dimethylamin, Diethylamin, Dipropylamin, Di-t-butylamin, Di-isobutylamin, Di-n-butylamin, Dicyclohexylamin, Dibenzylamin, Diphenylamin, Dioctylamine, Methylphenylamin, Ethylphenylamin oder Dihexylamin.

Das 1,4-Bis(chlor-disulfanyl)benzol lässt sich in guten Ausbeuten z.B. durch Umsetzung von Benzol mit Dichlordisulfan unter Verwendung von Lewis-Säuren-Katalysatoren erhalten. Geeignete Katalysatoren sind z.B. Montmorillonit ®KSF der Firma ACROS (Oberfläche nach BET 20-40 m$^2$/g) , Montmorillonit ®K10 (Oberfläche nach BET 220-270 m$^2$/g) oder auch Vulkasil® S/Aluminiumtrichlorid (Vulkasil S ist eine gefällte Kieselsäure der Bayer Material Science AG mit einer Oberfläche nach BET von 175 m$^2$/g).

Die erfindungsgemäßen organischen Schwefel-Stickstoff-Verbindungen der obigen Formel können z.B. als Vulkanisationsmittel für ungesättigte Kautschukmischungen zur Herstellung von GummiFormkörpern, wie z.B. Schläuche, Dichtungen, Motorlager und Fender, insbesondere aber zur Herstellung von Reifenbauteilen, wie z.B. Laufflächen, Drahtkappen, Seitenteil- und Wulststreifen, Schulterpolster, Gürtelabdeckungen, Laufflächen-Unterplatten und Seitenwände eingesetzt werden.

Die Herstellung der erfindungsgemäßen Kautschukmischungen erfolgt in an sich bekannter Weise durch übliches Vermischen der ungesättigten Kautschukkomponenten mit den bekannten Zusätzen (bzw. Zuschlagstoffen), wie Russ, Kieselsäure, Weichmacher, Alterungsschutzmittel, Zinkoxid, Stearinsäure, Harz, Verarbeitungswirkstoff sowie dem Vulkanisiersystem bestehend aus den erfindungsgemäßen neuen organischen Schwefel-Stickstoff-Verbindungen und gegebenenfalls zusätzlich elementarem Schwefel.

Die erfindungsgemäßen organischen Schwefel-Stickstoff-Verbindungen können entweder bei der Herstellung der Grundmischung unter sorgfältigem Ausschluss einer Anvulkanisation eingesetzt werden oder bevorzugt bei der Herstellung der Fertigmischung zusammen mit den Vulkanisationsbeschleunigern und dem Schwefel, falls erwünscht.

Ungesättigte Kautschuke im Sinne der Erfindung sind z.B. Naturkautschuk (NR), Isoprenkautschuk (IR), Butadienkautschuk (BR) und Styrol-Butadien-Kautschuk (SBR), der nach dem Emulsionsverfahren als auch nach dem Lösungsverfahren hergestellt sein kann, Nitrilkautschuk (NBR), teilhydrierter Nitrilkautschuk (H-NBR) und Ethylen-Propylen-Dienkautschuk (EPDM). Gleich gute Ergebnisse werden auch mit Verschnitten dieser Kautschuke erhalten.

Die Anwendung der Russe unterliegt keiner Einschränkung. Bevorzugt werden die in der kautschukverarbeitenden Industrie typischerweise angewendeten Russe eingesetzt, wie z.B. aktive und halbaktive Russe.

Die Anwendung der Kieselsäuren unterliegt ebenfalls keiner Einschränkung. Bevorzugt werden hochdisperse Kieselsäuren, hergestellt durch Fällung von Lösungen von Silikaten oder durch Flammhydrolyse von Siliciumhalogeniden. Die bevorzugten Kieselsäuren haben eine spezifische Oberfläche von 20 bis 400 m$^2$/g (BET-Oberfläche) und eine Primärteilchengröße von 10 bis 400 nm.

Die erfindungsgemäßen Schwefel-Stickstoff-Verbindungen werden in Mengen von ca. 0,2 bis 10 Gew.-Teilen, bevorzugt in Mengen von 0,5 bis 6,0 Gew.-Teilen, eingesetzt, bezogen auf 100 Gew.-Teile Kautschuk. Sofern zusätzlich Schwefel eingesetzt werden soll, kommt der üblicherweise in der kautschukverarbeitenden Industrie verwendete Schwefel oder auch unlöslicher Schwefel in Frage. Die bevorzugte Menge an Schwefel beträgt ca. 0,05 bis 2,5 Gew.-Teile, bevorzugt 0,1 bis 1,5 Gew.-Teile, bezogen auf 100 Gew.-Teile Kautschuk.

Selbstverständlich können anstelle des Schwefels auch die bekannten Schwefelspender, beispielsweise Caprolactamdisulfid, wie auch Abmischungen mit Schwefel eingesetzt werden. Die für den Verwendungszweck günstigste Menge an Schwefelspender kann durch Vorversuche leicht ermittelt werden.

Als Vulkanisationsbeschleuniger können die verschiedensten Typen eingesetzt werden und unterliegen keiner Einschränkung. Bevorzugt werden Mercaptobenzothiazol (MBT), Dibenzothiazyldisulfid (MBTS), Sulfenamide auf Basis von MBT, wie z.B. Benzothiazyl-2-cyclohexylsulfenamid (CBS), Benzothiazyl-2-dicyclohexylsulfen-amid (DCBS), Benzothiazyl-2-tert.-butylsulfenamid (TBBS) und Benzothiazyl-2-sulfenmorpholid (MBS) eingesetzt. Die Vulkanisationsbeschleuniger werden in Mengen von 0,5 bis 4,0 Gew.-Teilen, bevorzugt in Mengen von ca. 1,0 bis 3,5 Gew.-Teilen, bezogen auf 100 Gew.-Teile eingesetzten Kautschuk, eingesetzt. Es können aber auch Mischungen aus Vulkanisationsbeschleunigern eingesetzt werden, deren optimale Zusammensetzung hinsichtlich Typ und Menge leicht durch Versuche ermittelt werden kann.

Die Vulkanisation der erfindungsgemäßen Kautschukmischungen wird in bekannter Weise bei Temperaturen zwischen ca. 120° bis 220°C, bevorzugt von 140° bis 180°C, durchgeführt.

### Beispiele

Herstellung der erfindungsgemäßen organischen Schwefelverbindungen der Formel (I)

### Beispiel 1

Synthese von 1,4-Bis(diethylaminodisulfanyl)benzol

In einen inertisierten Dreihalskolben mit Tropftrichter, Rückflusskühler mit aufgesetzter Olive, einem Hahn mit Olive und einem Teflon-Rührfisch werden im Argongegenstrom 25,0 ml (190 mmol) Diethylamin in 150 ml 1,2-Dichlorethan vorgelegt. Dann werden unter Rühren 10,6 g (38,6 mmol) 1,4-Bis(chlor-disulfanyl)benzol in 50 ml 1,2-Dichlorethan langsam zugetropft. Die Reaktionsmischung wird vier Stunden bei Raumtemperatur gerührt. Das entstandene Salz wird abfiltriert, die organische Phase dreimal mit Wasser gewaschen und über Natriumsulfat getrocknet. Nach Entfernen des Lösungsmittels am Rotationsverdampfer und Trocknen im Drehschieberpumpenvakuum fällt 1,4-Bis(diethylaminodisulfanyl)benzol in Form eines gelben Öles an. Die Rohausbeute beträgt 11,9 g (89 %). Die Elementaranalyse wurde am Rohprodukt durchgeführt.

| C ber.: 48,23 | H ber.: 6,94 | N ber.: 8,04 | S ber.: 36,79 |
|---|---|---|---|
| C gef.: 44,88 | H gef.: 5,62 | N gef.: 6,33 | S gef.: 34,17 |

### Beispiel 2

Synthese von 1,4-Bis(*t*-butylaminodisulfanyl)benzol

In einen inertisierten Dreihalskolben mit Tropftrichter, Rückflusskühler mit aufgesetzter Olive, einem Hahn mit Olive und einem Teflon-Rührfisch werden im Argongegenstrom 100 ml (941 mmmol) *t*-Butylamin in 1,2-Dichlorethan vorgelegt. Dann werden unter Rühren 10,6 ml (38,6 mmol) 1,4-Bis(chlor-disulfanyl)benzol in 50 ml 1,2-Dichlorethan zugetropft. Die Reaktionsmischung wird vier Stunden bei Raumtemperatur gerührt. Das entstandene Salz wird abfiltriert, die organische Phase dreimal mit Wasser gewaschen und über Natriumsulfat getrocknet. Nach Entfernen des Lösungsmittels am Rotationsverdampfer und Trocknen im Drehschieberpumpenvakuum fällt 1,4-Bis(*t*-butylaminodisulfanyl)benzol in Form eines gelben Öles an. Die Rohausbeute beträgt 11,9 g (88 %). Die Elementaranalyse wurde am Rohprodukt durchgeführt.

| C ber.: 48,23 | H ber.: 6,94 | N ber.: 8,04 | S ber.: 36,79 |
|---|---|---|---|
| C gef.: 40,74 | H gef.: 4,88 | N gef.: 5,74 | S gef.: 44,10 |

### Beispiel 3

Synthese von α-*t*-Butylamino-ω-hydropoly[1-disulfanyl-4-(*t*-butylaminodisulfanyl)-phenylen]

In einen inertisierten Dreihalskolben mit Tropftrichter, Rückflusskühler mit aufgesetzter Olive, einem Hahn mit Olive und einem Teflon-Rührfisch werden im Argongegenstrom 11,7 g (42,4 mmol) 1,4-Bis-(chlordisulfanyl)benzol in 1,2-Dichlorethan vorgelegt. Dann wird unter Rühren 13,4 g (125 mmol) *t*-Butylamin zugetropft. Die Reaktionsmischung werden vier Stunden bei Raumtemperatur gerührt. Das entstandene Salz wird abfiltriert, die organische Phase dreimal mit Wasser gewaschen und über Natriumsulfat getrocknet. Nach Entfernen des Lösungsmittels am Rotationsverdampfer und Trocknen im Drehschieberpumpenvakuum fällt α-*t*-Butyl-amino-ω-hydropoly[1-disulfanyl-4-(*t*-butylaminodisulfanyl)phenylen] in Form eines gelben Harzes an. Die Rohausbeute beträgt 12,7 g (99 %). Die Elementaranalyse wurde am Rohprodukt durchgeführt.

| C ber.: 43,60 | H ber.: 4,76 | N ber.: 5,08 | S ber.: 46,56 |
|---|---|---|---|
| C gef.: 36,88 | H gef.: 4,46 | N gef.: 5,32 | S gef.: 36,61 |

### Beispiel 4

Synthese von 1,6-Bis(diethylaminotrisulfanyl)hexan

In einen inertisierten Dreihalskolben mit Tropftrichter, Rückflusskühler mit aufgesetzter Olive, einem Hahn mit Olive und einem Teflon-Rührfisch werden im Argongegenstrom 20,0 ml (152 mmol) Diethylamin in 1,2-Dichlorethan vorgelegt. Dann werden unter Rühren 10,4 g (29,7 mmol) 1,6-Bis(chlor-trisulfanyl)hexan in 50 ml 1,2-Dichlorethan langsam zugetropft. Die Reaktionsmischung wird vier Stunden bei Raumtemperatur gerührt. Das entstandene Salz wird abfiltriert, die organische Phase dreimal mit Wasser gewaschen und über Natriumsulfat getrocknet. Nach Entfernen des Lösungsmittels am Rotationsverdampfer und Trocknen im Drehschieberpumpenvakuum fällt 1,6-Bis(diethylaminotrisulfanyl)hexan in Form eines gelben Öles an. Die Rohausbeute beträgt 5,32 g (82%). Die Elementaranalyse wurde am Rohprodukt durchgeführt.

| C ber.: 39,96 | H ber.: 7,6 | N ber.: 6,66 | S ber.: 45,72 |
|---|---|---|---|
| C gef.: 39,33 | H gef.: 7,63 | N gef.: 5,75 | S gef.: 45,97 |

**Beispiel 5**

Synthese von 1,6-Bis(*t*-butylaminotrisulfanyl)hexan

In einen inertisierten Dreihalskolben mit Tropftrichter, Rückflusskühler mit aufgesetzter Olive, einem Hahn mit Olive und einem Teflon-Rührfisch werden im Argongegenstrom 70,0 ml (736 mmol) *t*-Butylamin in 1,2-Dichlorethan vorgelegt. Dann werden unter Rühren 10,2 g (29,3 mmol) 1,6-Bis(chlor-trisulfanyl)hexan in 50 ml 1,2-Dichlorethan zugetropft. Die Reaktionsmischung wird vier Stunden bei Raumtemperatur gerührt. Das entstandene Salz wird abfiltriert, die organische Phase dreimal mit Wasser gewaschen und über Natriumsulfat getrocknet. Nach Entfernen des Lösungsmittels am Rotationsverdampfer und Trocknen im Drehschieber-pumpenvakuum fällt 1,6-Bis(*t*-butylaminotrisulfanyl)hexan in Form eines gelben Öles an. Die Rohausbeute beträgt 11,9 g (97%). Die Elementaranalyse wurde am Rohprodukt durchgeführt.

| C ber.: 39,96 | H ber.: 7,67 | N ber.: 6,66 | S ber.: 45,72 |
| C gef.: 38,82 | H gef.: 7,46 | N gef.: 5,15 | S gef.: 45,87 |

**Beispiel 6**

Synthese von α-*t*-Butylamino-ω-hydropoly[1-trisulfanyl-4-(*t*-butylaminotrisulfanyl)-hexandiyl]

In einen inertisierten Dreihalskolben mit Tropftrichter, Rückflusskühler mit aufgesetzter Olive, einem Hahn mit Olive und einem Teflon-Rührfisch werden im Argongegenstrom 11,2 g (32,2 mmol) 1,6-Bis-(chlortrisulfanyl)hexan in 150 ml 1,2-Dichlorethan vorgelegt. Dann werden unter Rühren 10,0 ml (94,7 mmol) *t*-Butylamin in 50 ml 1,2-Dichloethan zugetropft. Die Reaktionsmischung wird vier Stunden bei Raumtemperatur gerührt. Das entstandene Salz wird abfiltriert, die organische Phase dreimal mit Wasser gewaschen und über Natriumsulfat getrocknet. Nach Entfernen des Lösungsmittels am Rotationsverdampfer und Trocknen im Drehschieberpumpenvakuum fällt α-*t*-Butylamino-ω-hydropoly[1-trisulfanyl-4-(*t*-butylaminotrisulfanyl)hexandiyl] in Form eines gelben Harzes an. Die Ausbeute beträgt 64,4 g (99 %). Die Elementaranalyse wurde am Rohprodukt durchgeführt.

| C ber.: 34,55 | H ber.: 6,09 | N ber.: 4,03 | S ber.: 55,34 |
| C gef.: 34,70 | H gef.: 6,20 | N gef.: 3,34 | S gef.: 53,63 |

**Beispiel 7**

Snythese von α-Cyclohexylamino-ω-hydrooligo[1-disulfanyl-4-(disulfanylcyclohexylamino)phenylen]

In einen inertisierten Dreihalskolben mit Tropftrichter, Rückflusskühler mit aufgesetzter Olive, einem Hahn mit Olive und einem Teflon-Rührfisch werden im Argongegenstrom 375,0 ml (3,4 mol) Cyclohexylamin in 100 ml Tetrachlormethan vorgelegt. Dann werden unter Rühren 51,3 g (190 mmol) 1,4-Bis(chlor-disulfanyl)benzol in 50 ml Tetrachlormethan langsam zugetropft. Die Reaktionsmischung wird 36 Stunden bei Raumtemperatur gerührt. Das entstandene Salz wird abfiltriert, die organische Phase dreimal mit Wasser gewaschen und über Natriumsulfat getrocknet. Nach Entfernen des Lösungsmittels am Rotationsverdampfer und Trocknen im Drehschieberpumpenvakuum fällt α-Cyclohexylamino-ω-hydrooligo[1-disulfanyl-4-(disulfanylcyclohexylamino)-phenylen] als zähes, klebrig braunes Öl an. Die Rohausbeute beträgt 65,8 g (86 %).

**Beispiel 8**

Synthese von Bis(diethylaminooligosulfanyl)hexan durch Polymerabbau

In einen 500 ml-Rundkolben mit Rückflusskühler mit aufgesetztem Calcium-chloridtrockenrohr werden 100 ml (760 mmol) Diethylamin und 2,73 g (12,9 mmol) Poly(1,6-tetrathiohexandiyl) in 200 ml Chloroform gegeben. Dann wird die Reaktionsmischung unter Rühren fünf Tage im Rückfluss gehalten. Das entstandene Salz wird abfiltriert, die organische Phase dreimal mit Wasser gewaschen und über Natriumsulfat getrocknet. Nach Entfernen des Lösungsmittels am Rotationsverdampfer und Trocknen im Drehschieberpumpenvakuum fallen 3,52 g (93 %) 1,6-Bis(diethylaminooligosulfanyl)hexan in Form eines dunkelroten Öles an.

Erläuterungen zu den gummitechnologischen Prüfungen

Folgende Testmethoden bzw. Testvorrichtungen wurden benützt: Rheometer: ASTM D 2084, Monsanto MDR 2000E. Zugversuch: DIN 53405, S2-Stab. Härte: DIN 53505. Rückprallelastizität: DIN 53512. Weiterreißwiderstand: DIN 53504. Abrieb DIN 53516. Viskoelastische Eigenschaften: DIN 53513/ISO 4664, Roelig-Test 10 Hz. Dynamische Eigenschaften: DIN 53533.

**Beispiel 9**

Die organischen Vulkanisiermittel wurden in einer russgefüllten Naturkautschukmischung (100 Teile NR, 5 Teile ZnO RS, 1 Teil Stearinsäure, 50 Teile Russ N375, 6 Teile 6PPD, 1 Teil TMQ, 1 Teil Ozonschutzwachs, 3 Teile aromatischer Weichmacher) getestet. Die Herstellung erfolgte im Innenmischer GK 1,5E der Fa. Werner & Pfleiderer. Schwefel, Beschleuniger (CBS) und Vulkanisiermittel wurden auf der Walze nachgemischt. Die Mengenangaben stellen Gew.-Teile pro 100 Gew.-Teile Kautschuk dar.

Tabelle 1:

| Vernetzerdosierung | |
|---|---|
| Vergleich 1 | 1 Teil Schwefel |
| Polyphenylensulfid | 3 Teile Polyphenylensulfid |
| Beispiel 1 | 1,26 Teile Verbindung gemäß Beispiel 1, 0,57 Teile Schwefel |
| Beispiel 2 | 1,57 Teile Verbindung gemäß Beispiel 2, 0,49 Teile Schwefel |
| Beispiel 3 | 1,26 Teile Verbindung gemäß Beispiel 3, 0,57 Teile Schwefel |
| Beispiel 4 | 1,86 Teile Verbindung gemäß Beispiel 4, 0,36 Teile Schwefel |
| Beispiel 5 | 1,86 Teile Verbindung gemäß Beispiel 5, 0,36 Teile Schwefel |
| Beispiel 6 | 1,59 Teile Verbindung gemäß Beispiel 6, 0,36 Teile Schwefel |

Als Beschleuniger wurde zu jeder Mischung 3,4 Teile CBS zugegeben.

Tabelle 2:

| Bestimmung der Reversion im Rheometer | | |
|---|---|---|
| | Drehmoment relativ zum Vergleich 1 | Reversion relativ zum Vergleich 1 |
| Vergleich 1 | 100 % | 100 % |
| Polyphenylensulfid | 12 % | -- |
| Beispiel 1 | 97 % | 75 % |
| Beispiel 2 | 99 % | 47 % |
| Beispiel 3 | 99 % | 61 % |
| Beispiel 4 | 107 % | 8 % |
| Beispiel 5 | 111 % | 3 % |
| Beispiel 6 | 106 % | 5 % |

Die Reversion in % wurde aus den Rheometer-Daten (160 °C, 60 min) wie folgt bestimmt:

$$(S'max - S'end) \times 1/S'max \times 100 \ (\%)$$ und relativ zum Vergleich 1 berechnet.

Aus Tabelle 2 geht hervor, dass einmal die Mischungen gemäß der Erfindung eine deutlich höhere Reversionsbeständigkeit (je kleiner der Wert der Reversion, desto höher die Reversionsbeständigkeit) aufweisen als die Vergeichsmischung 1. Zum anderen weist Polyphenylensulfid praktisch keine Vernetzungsaktivität auf, eine Berechnung der Reversionsbeständigkeit ergibt deshalb keinen Sinn.

**Beispiel 10**

Mit Hilfe eines Innenmischers vom Typ GK 1,5E der Firma Werner & Pfleiderer wurden bei einer Rotordrehzahl von 40 upm und einer Kammer- und Schaufeltemperatur von 50°C (Stempeldruck 8 bar, Füllgrad 65%) Testmischungen gemäß Tabelle 3 hergestellt.

Beschleuniger, Schwefel und die neuen Schwefelverbindungen gemäß Beispiel 7 wurden auf der Walze zugemischt. Die Mengenangaben stellen Gew.-Teile pro 100 Gew.-Teile Kautschuk dar.

Tabelle 3:

| Testrezeptur | | |
|---|---|---|
| | Vergleich | Erfindung |
| Mischungsnummer | 1 | 2 |
| NR (TSR 5, Defo 700) | 100 | 100 |
| Zinkoxid RS | 5 | 5 |
| Stearinsäure | 1 | 1 |
| Russ N375 | 50 | 50 |
| 6PPD | 2 | 2 |
| TMQ | 1 | 1 |
| Ozonschutzwachs | 1 | 1 |
| Arom. Weichmacher | 3 | 3 |
| Schwefel | 1,5 | 0,5 |
| CBS | 1,5 | 1,5 |
| Schwefelverbindung gemäß Beispiel 7 (Erfindung) | 0 | 3 |

Tabelle 4:

| Rheometer-Daten | | |
|---|---|---|
| | Vergleich | Erfindung |
| Mischungsnummer | 1 | 2 |
| 150°C/60 min | | |
| t01 (min) | 2,75 | 1,1 |
| t90 (min) | 5,67 | 4,17 |
| S' max (dNm) | 22,19 | 19,62 |
| S' end (dNm) | 19,91 | 19,33 |
| Reversion (%) | 10,3 | 1,5 |

Die Reversion in % wurde aus den Rheometer-Daten wie folgt bestimmt:

$$(S'max - S'end) \times 1/S'max \times 100 \ (\%)$$

Aus Tabelle 4 geht hervor, dass einmal die Mischungen gemäß der Erfindung eine deutlich höhere Reversionsbeständigkeit (je kleiner der Wert der Reversion, desto höher die Reversionsbeständigkeit) aufweisen als die Vergleichsmischung 1.

Tabelle 5:

| Eigenschaften der Testvulkanisate nach Optimalheizung | | |
|---|---|---|
| | Vergleich | Erfindung |
| Mischungsnummer | 1 | 2 |
| Vulkanisation: 150°C/11 min | | |
| Festigkeit (MPa) | 32 | 32 |
| Bruchdehnung (%) | 555 | 579 |
| Modul 100 (MPa) | 2,6 | 2,5 |
| Modul 300 (MPa) | 13,8 | 13,0 |

Tabelle 5:   (fortgesetzt)

| Eigenschaften der Testvulkanisate nach Optimalheizung | | |
|---|---|---|
| | Vergleich | Erfindung |
| Weiterreißfestigkeit (N) | 64 | 61 |
| Härte bei 23°C (Shore A) | 65 | 65 |
| Elastizität bei 23°C (%) | 44 | 44 |
| Abrieb (mm$^3$) | 109 | 122 |
| | | |
| Roelig 0°C | | |
| tan delta | 0,232 | 0,215 |
| E' (MPa) | 11,036 | 1.1,954 |
| E" (MPa) | 2,562 | 2,570 |
| Roelig 60°C | | |
| tan delta | 0,125 | 0,137 |
| E' (MPa) | 6,675 | 7,015 |
| E" (MPa) | 0,833 | 0,959 |

Aus Tabelle 5 geht hervor, dass die Vulkanisate gemäß der Erfindung vergleichbare Eigenschaften nach Optimalheizung aufweisen wie das Vergleichsvulkanisat.

Tabelle 6:

| Eigenschaften der Testvulkanisate nach Überheizung | | |
|---|---|---|
| | Vergleich | Erfindung |
| Mischungsnummer | 1 | 2 |
| | | |
| Vulkanisation: 160°C/120 min | | |
| Festigkeit (MPa) | 25,2 | 26,8 |
| Bruchdehnung (%) | 547 | 518 |
| Modul 100 (MPa) | 2,0 | 2,4 |
| Modul 300 (MPa) | 10,5 | 13,0 |
| Weiterreißfestigkeit (N) | 34 | 45 |
| Härte bei 23°C (Shore A) | 63 | 62 |
| Elastizität bei 23°C (%) | 45 | 41 |
| Abrieb (mm$^3$) | 228 | 158 |
| | | |
| Roelig 0°C | | |
| tan delta | 0,249 | 0,230 |
| E'(MPa) | 10,194 | 12,500 |
| E" (MPa) | 2,538 | 2,876 |
| | | |
| Roelig 60°C | | |
| tan delta | 0,150 | 0,149 |
| E' (MPa) | 5,587 | 6,683 |
| E" (MPa) | 0,835 | 0,995 |

Aus Tabelle 6 geht hervor, dass nach Überheizung die Vulkanisate gemäß der Erfindung eine günstigere Retention des Moduls, Festigkeit, Weiterreißfestigkeit und Abrieb aufweisen als das Vergleichsvulkanisat.

**Patentansprüche**

1. Organische Schwefel-Stickstoff-Verbindungen der Formel

$$R^1R^2N(-S_x-R^3-S_x-N\,R^4)_n-S_x-R^3-S_x-NR^5R^6,$$

worin

R$^1$, R$^2$, R$^5$ und R$^6$      gleich oder verschieden sind und für Wasserstoff, Methyl, Ethyl, n-Butyl, iso-Butyl, t-Butyl, Hexyl, Cyclohexyl, Octyl, Phenyl oder Benzyl stehen,

R$^4$      Methyl, Ethyl, n-Butyl, iso-Butyl, t-Butyl, Hexyl, Cyclohexyl, Octyl, Phenyl oder Benzyl bedeutet,

n      eine ganze Zahl von 1 bis 50 ist, mit der Maßgabe, dass n = 0 ist, wenn R$^1$, R$^2$, R$^5$ oder R$^6$ ungleich Wasserstoff ist,

R$^3$      für Phenylen, Methylen, Ethylen, Butylen oder Hexylen steht und

x      eine ganze Zahl von 2 bis 6 darstellt.

2. Verfahren zur Herstellung der organischen Schwefel-Stickstoff-Verbindungen gemäß Anspruch 1 durch Umsetzung von entsprechenden Aminen mit Bis(dichlorpolysulfanyl)-arylenen oder - alkylenen in inerten Lösungsmitteln.

3. Verwendung der organischen Schwefel-Stickstoff-Verbindungen gemäß Anspruch 1 als Vulkanisiermittel zur Herstellung von ungesättigten Kautschukvulkanisaten.

4. Verwendung der Schwefel-Stickstoff-Verbindungen gemäß Anspruch 1 zur Herstellung von Gummiformkörpern.

5. Verwendung der Schwefel-Stickstoff-Verbindungen gemäß Anspruch 1 zur Herstellung von Reifenbauteilen.

**Europäisches Patentamt**

## EUROPÄISCHER RECHERCHENBERICHT

Nummer der Anmeldung

EP 05 00 6050

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| A,D | EP 0 530 590 A (HUELS AKTIENGESELLSCHAFT) 10. März 1993 (1993-03-10) * Seite 3, Zeile 39 - Seite 4, Zeile 13 * * Seite 5, Zeile 17 - Zeile 20 * * Seite 5, Zeile 28 - Zeile 37 * * Seite 7, Zeile 6 - Zeile 12; Ansprüche 1,5; Beispiele * | 1-5 | C08K5/372 C08G75/14 C08J3/24 C07C319/22 C07C323/30 |
| A | HELT W F ET AL: "POST VULCANIZATION STABILIZATION FOR NR" RUBBER WORLD, LIPPINCOTT & PETO, AKRON, OH, US, Bd. 204, Nr. 5, Januar 1991 (1991-01), Seiten 18-24, XP000610653 ISSN: 0035-9572 * Seite 18 - Seite 19; Tabellen 2,5,7,9 * * Seite 21 * * Seite 23 - Seite 24 * | 1-5 | |
| A | US 2 490 518 A (HAND JAMES F) 6. Dezember 1949 (1949-12-06) * Spalte 1, Zeile 1 - Zeile 4 * * Spalte 1, Zeile 19 - Zeile 22 * * Spalte 1, Zeile 51 - Spalte 2, Zeile 28 * * Spalte 4, Zeile 59 - Zeile 70; Ansprüche 1,2; Tabelle I * | 1-5 | **RECHERCHIERTE SACHGEBIETE (Int.Cl.7)** C08K C08G C08J C07C |
| A | PATENT ABSTRACTS OF JAPAN Bd. 1998, Nr. 02, 30. Januar 1998 (1998-01-30) & JP 09 278942 A (SUMITOMO RUBBER IND LTD), 28. Oktober 1997 (1997-10-28) * Zusammenfassung * | 1-5 | |

-/--

Der vorliegende Recherchenbericht wurde für alle Patentansprüche erstellt

| Recherchenort | Abschlußdatum der Recherche | Prüfer |
|---|---|---|
| München | 19. August 2005 | Seelmann, M |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.....................................................................................
& : Mitglied der gleichen Patentfamilie, übereinstimmendes Dokument

EPO FORM 1503 03.82 (P04C03)

**Europäisches Patentamt**

**EUROPÄISCHER RECHERCHENBERICHT**

Nummer der Anmeldung

EP 05 00 6050

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| A | PATENT ABSTRACTS OF JAPAN Bd. 2000, Nr. 19, 5. Juni 2001 (2001-06-05) & JP 2001 031797 A (YOKOHAMA RUBBER CO LTD:THE), 6. Februar 2001 (2001-02-06) * Zusammenfassung * ----- | 1-5 | |

RECHERCHIERTE SACHGEBIETE (Int.Cl.7)

Der vorliegende Recherchenbericht wurde für alle Patentansprüche erstellt

| Recherchenort | Abschlußdatum der Recherche | Prüfer |
|---|---|---|
| München | 19. August 2005 | Seelmann, M |

KATEGORIE DER GENANNTEN DOKUMENTE

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
   nach dem Anmeldedatum veröffentlicht worden ist
D : in der Anmeldung angeführtes Dokument
L : aus anderen Gründen angeführtes Dokument
.......................................................................................................
& : Mitglied der gleichen Patentfamilie, übereinstimmendes
   Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 05 00 6050

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

19-08-2005

| Im Recherchenbericht angeführtes Patentdokument | Datum der Veröffentlichung | Mitglied(er) der Patentfamilie | Datum der Veröffentlichung |
|---|---|---|---|
| EP 0530590 A | 10-03-1993 | DE 4128869 A1<br>DE 4225684 A1<br>AT 133189 T<br>CA 2077156 A1<br>DE 59205083 D1<br>EP 0530590 A1<br>ES 2082303 T3<br>JP 3142653 B2<br>JP 5209092 A<br>US 5342900 A | 04-03-1993<br>10-02-1994<br>15-02-1996<br>01-03-1993<br>29-02-1996<br>10-03-1993<br>16-03-1996<br>07-03-2001<br>20-08-1993<br>30-08-1994 |
| US 2490518 A | 06-12-1949 | KEINE | |
| JP 09278942 A | 28-10-1997 | JP 3157103 B2 | 16-04-2001 |
| JP 2001031797 A | 06-02-2001 | DE 10008641 A1<br>FR 2790262 A1<br>FR 2845092 A1<br>FR 2845093 A1<br>US 2003195302 A1<br>US 2003204026 A1<br>US 6518367 B1 | 31-08-2000<br>01-09-2000<br>02-04-2004<br>02-04-2004<br>16-10-2003<br>30-10-2003<br>11-02-2003 |

EPO FORM P0461

Für nähere Einzelheiten zu diesem Anhang : siehe Amtsblatt des Europäischen Patentamts, Nr.12/82